# EUROPEAN PATENT SPECIFICATION

(11) **EP 2 473 482 B1**
(45) Date of publication and mention of the grant of the patent: **05.03.2014**
(21) Application number: 10814409.8
(22) Date of filing: 01.09.2010
(51) Int. Cl.: A61K 31/445, C07D 211/30, A61P 31/16

(54) **METHODS OF TREATING ORTHOMYXOVIRAL INFECTIONS**
VERFAHREN ZUR BEHANDLUNG VON ORTHOMYXOVIRUSINFEKTIONEN
MÉTHODES DE TRAITEMENT D'INFECTIONS ORTHOMYXOVIRALES

(30) Priority: 11.06.2010 US 353935 P; 22.02.2010 US 282508 P; 04.09.2009 US 272254 P
(43) Date of publication of application: 11.07.2012
(73) Proprietor: United Therapeutics Corporation, Silver Spring, MD 20910 (US); THE CHANCELLOR, MASTERS AND SCHOLARS OF THE UNIVERSITY OF OXFORD, Oxford OX1 2JD (GB)
(72) Inventor: RAMSTEDT, Urban, Silver Spring, MD 20910 (US); KLOSE, Brennan, Silver Spring, MD 20910 (US); ZITZMANN, Nicole, Oxford OX1 4TL (GB); DWEK, Raymond, A., Oxford OX2 9AU (GB); BUTTERS, Terry, D., Oxford OX44 9BS (GB)
(74) Representative: HOFFMANN EITLE
(86) International application number: PCT/US2010/047488
(87) International publication number: WO 2011/028775

(56) References cited:
- WO-A1-95/22975
- US-A1- 2007 275 998
- US-A1- 2008 138 351
- US-A1- 2008 138 351
- US-B2- 7 256 005
- ZENG YUCHENG ET AL: "Homonojirimycin and N-methyl-homonojirimycin inhibit N-linked oligosaccharide processing", GLYCOBIOLOGY, vol. 7, no. 2, 1997, pages 297-304, XP002688520, ISSN: 0959-6658
- BOSCH F X ET AL: "Effect of inhibitors of glycosylation on proteolytic activation of avian influenza virus hemagglutinins: Discrimination between tryptic cleavage and elimination of the connecting peptide", VIROLOGY, ACADEMIC PRESS,ORLANDO, US, vol. 132, no. 1, 15 January 1984 (1984-01-15), pages 199-204, XP023047753, ISSN: 0042-6822, DOI: 10.1016/0042-6822(84)90103-X [retrieved on 1984-01-15]
- GU BAOHUA ET AL: "Antiviral profiles of novel iminocyclitol compounds against bovine viral diarrhea virus, West Nile virus, dengue virus and hepatitis B virus", ANTIVIRAL CHEMISTRY & CHEMOTHERAPY, BLACKWELL SCIENTIFIC PUBL., LONDON, GB, vol. 18, no. 1, 1 January 2007 (2007-01-01), pages 49-59, XP008120632, ISSN: 0956-3202
- JORDAN R ET AL: "Inhibition of Host ER Glucosidase Activity Prevents Golgi Processing of Virion-Associated Bovine Viral Diarrhea Virus E2 Glycoproteins and Reduces Infectivity of Secreted Virions", VIROLOGY, ACADEMIC PRESS,ORLANDO, US, vol. 295, no. 1, 30 March 2002 (2002-03-30), pages 10-19, XP004467139, ISSN: 0042-6822, DOI: 10.1006/VIRO.2002.1370
- RATNER L ET AL: "Inhibition of HIV and SIV infectivity by blockade of alpha-glucosidase activity", VIROLOGY, ACADEMIC PRESS,ORLANDO, US, vol. 181, no. 1, 1 March 1991 (1991-03-01) , pages 180-192, XP023057038, ISSN: 0042-6822, DOI: 10.1016/0042-6822(91)90483-R [retrieved on 1991-03-01]
- NATURE REVIEWS DRUG DISCOVERY, vol. 1, no. 1, 1 January 2002 (2002-01-01) , pages 65-75,
- ARCHIVES OF VIROLOGY, vol. 81, 1984, pages 25-39,
- ANTIVIRAL RESEARCH, vol. 68, 2005, pages 163-172,
- ANTIVIRAL RESEARCH, vol. 68, 2005, pages 1-9,
- ANTIVIRAL RESEARCH, vol. 65, 2005, pages 125-131,
- CHEMICAL AND PHARMACEUTICAL BULLETIN, vol. 49, no. 11, 2001, pages 1471-1473,
- MICROBIOLOGY AND IMMUNOLOGY, vol. 45, no. 7, 2001, pages 531-537,
- ANTIVIRAL RESEARCH, vol. 14, 1990, pages 215-226,

## Description

### RELATED APPLICATIONS

The present application claims priority to a) US provisional application no. 61/272,254 filed September 4, 2009; b) US provisional application no. 61/282,508 filed February 22, 2010 and c) US provisional application no. 61/353,935 filed June 11, 2010.

### FIELD

The present application relates to iminosugars and methods of treating viral infections with iminosugars and, in particular, to the use of iminosugars for treatment and/or prevention of viral infections caused by or associated with a virus belonging to the Orthomyxoviridae family.

### SUMMARY

One embodiment relates to a method of treating or preventing a disease or condition caused by or associated with a virus belonging to the Orthomyxoviridae family, which method comprises administering to a subject in need thereof an effective amount of a compound of the formula: 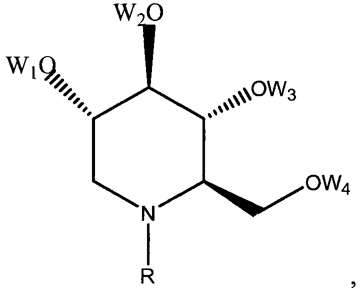 or a pharmaceutically acceptable salt thereof, wherein R is either selected from substituted or unsubstituted cycloalkyl groups, substituted or unsubstituted aryl groups, or substituted or unsubstituted oxaalkyl groups; or wherein R is 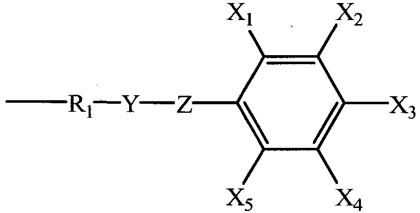
R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl; and
wherein W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

Another embodiment relates to a method of inhibiting infectivity of a cell infected with a virus belonging to the Orthomyxoviridae family, which method comprises contacting a cell infected with a virus belonging to the Orthomyxoviridae family with an effective amount of a compound of the formula: 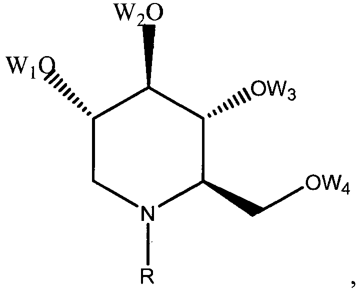 or a pharmaceutically acceptable salt thereof, wherein R is either selected from substituted or unsubstituted cycloalkyl groups, substituted or unsubstituted aryl groups, or substituted or unsubstituted oxaalkyl groups; or wherein R is 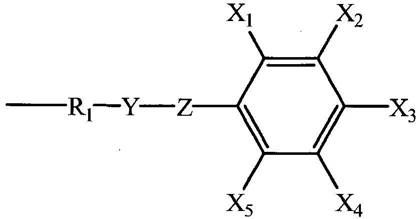
R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl; and
wherein W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

### DRAWINGS

Figures 1(A)-(E) present chemical formulas of the following iminosugars: A) *N*-Butyl deoxynojirimycin (NB-DNJ or UV-1); B) *N*-Nonyl deoxynojirimycin (NN-DNJ or UV-2); C) *N*-(7-Oxadecyl)deoxynojirimycin (N7-O-DNJ or UV-3); D) *N*-(9-Methoxynonyl) deoxynojirimycin (N9-DNJ or UV-4); E) *N*-(*N*-{4'-azido-2'-nitrophenyl}-6-aminohexyl)deoxynojirimycin (NAP-DNJ or UV-5).

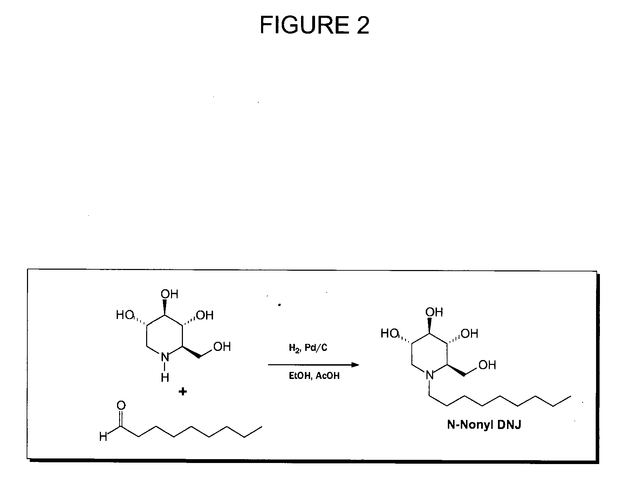

Figure 2 is a synthesis scheme for NN-DNJ.

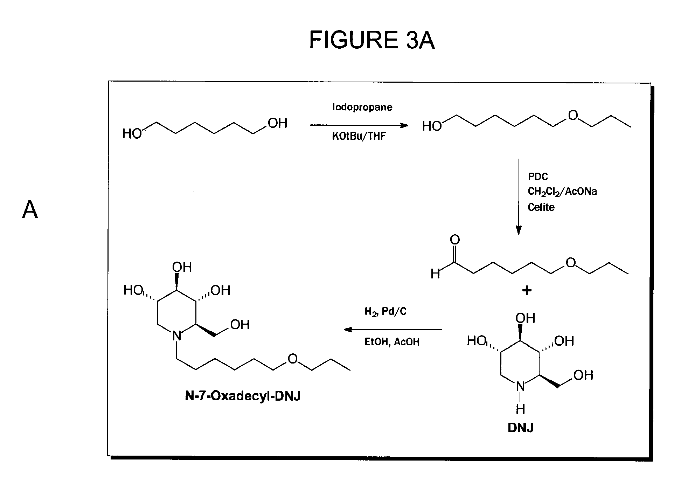

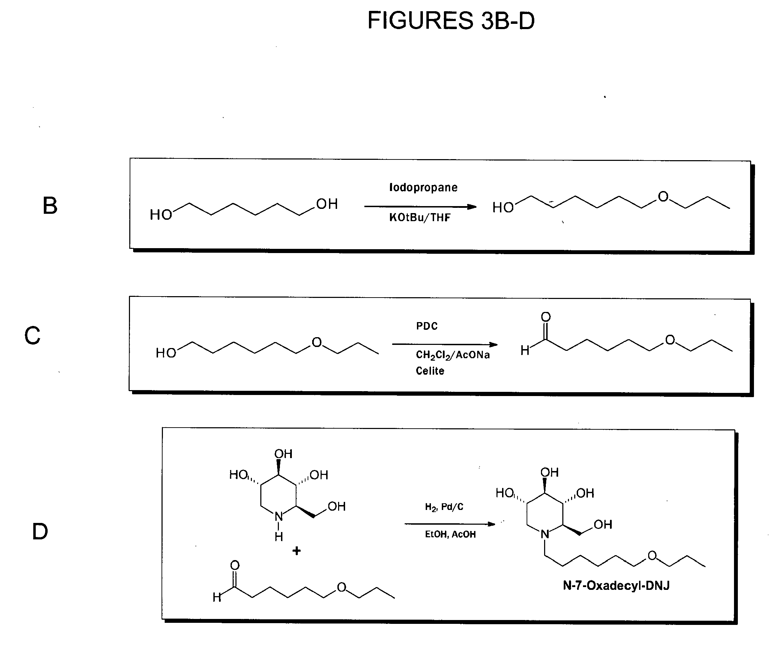

Figures 3A-D illustrate synthesis of N7-O-DNJ. In particular, Figure 3A shows a sequence of reactions leading to N7-O-DNJ; Figure 3B illustrates preparation of 6-propyloxy-1-hexanol; Figure 3C illustrates preparation of 6-propyloxy-1-hexanal; Figure 3D illustrates synthesis of N7-O-DNJ.
Figures 4A-C relate to synthesis of *N*-(9-Methoxynonyl) deoxynojirimycin. In particular,

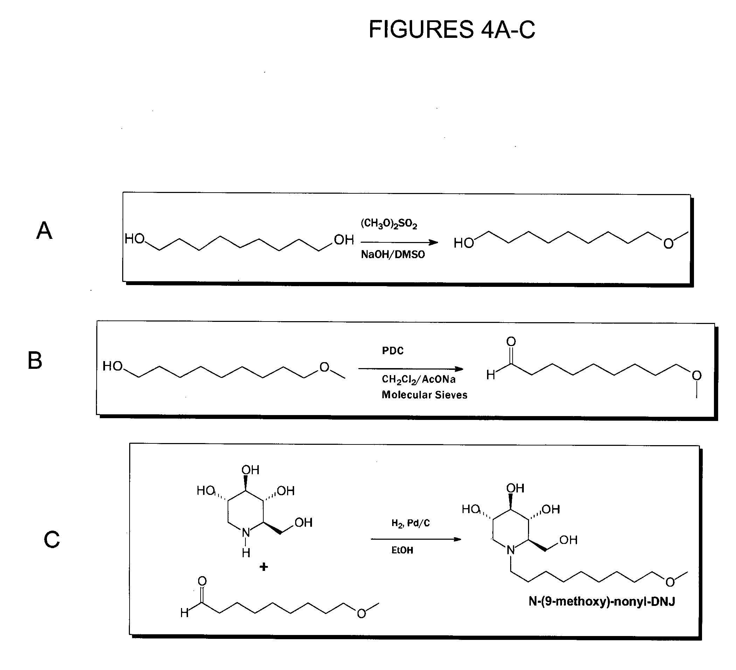

Figure 4A illustrates preparation of 9-methoxy-1-nonanol; Figure 4B illustrates preparation of 9-methoxy-1-nonanal; Figure 4C illustrates synthesis of *N*-(9-Methoxynonyl) deoxynojirimycin.

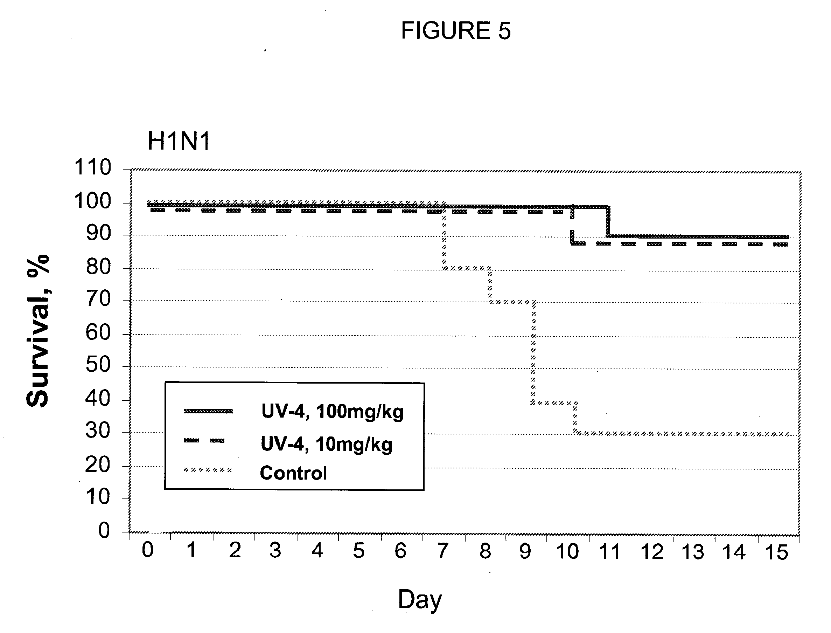

Figure 5 presents effects of 10 day administration of UV-5 on survival of mice infected with influenza A H1N1.

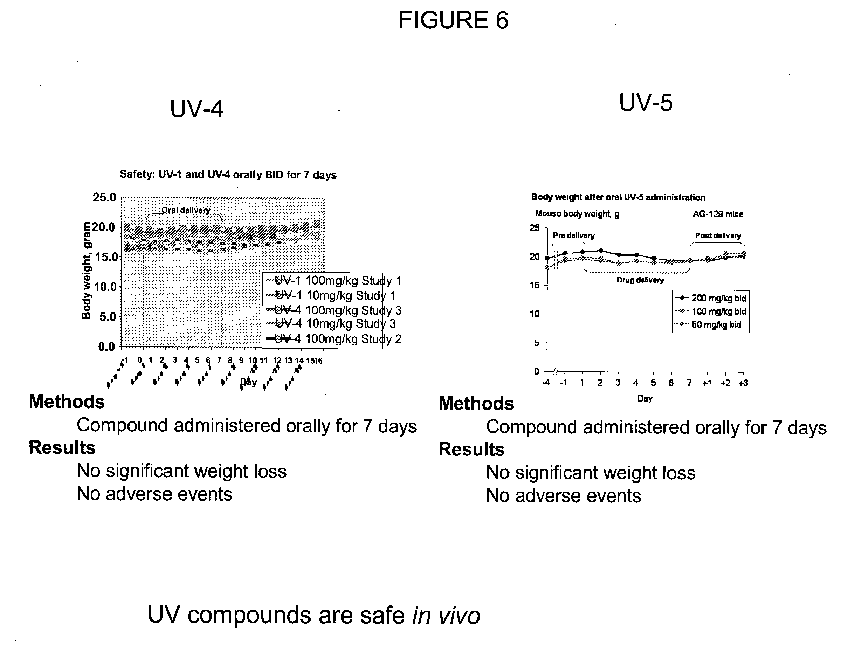

Figure 6 presents *in vivo* safety data for UV-4 and UV-5.

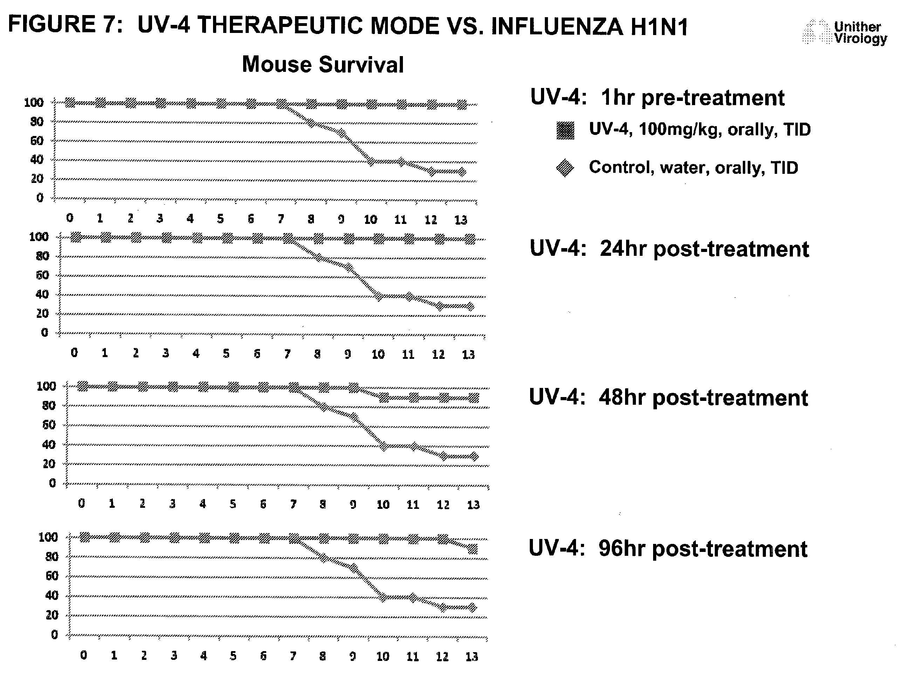

Figure 7 presents survival data after H1/N1 virus challenge for mice treated with UV-4 versus control mice.

### DETAILED DESCRIPTION

### Related Patent Documents

The following patent documents, may be useful for understanding the present disclosure:
1) US patent no. 6,545,021;
2) US patent no. 6,809,803;
3) US patent no. 6,689,759;
4) US patent no. 6,465,487;
5) US patent no. 5,622,972;
6) US patent application no. 12/656,992 filed February 22, 2010;
7) US patent application no. 12/656,993 filed February 22, 2010;
8) US patent application no. 12/813,882 filed June 11, 2010;
9) US patent provisional application no. 61/282,507 filed February 22, 2010;
10) US patent provisional application no. 61/272,252 filed September 4, 2009;
11) US provisional application no. 61/272,253 filed September 4, 2009;
12) US provisional application no. 61/272,254 filed September 4, 2009;
13) US provisional application no. 61/282,508 filed February 22, 2010;
14) US provisional application no. 61/353,935 filed June 11, 2010.

### Definition of terms

Unless otherwise specified, "a" or "an" means "one or more."

As used herein, the term "viral infection" describes a diseased state, in which a virus invades a healthy cell, uses the cell's reproductive machinery to multiply or replicate and ultimately lyse the cell resulting in cell death, release of viral particles and the infection of other cells by the newly produced progeny viruses. Latent infection by certain viruses is also a possible result of viral infection.

As used herein, the term "treating or preventing viral infection" means to inhibit the replication of the particular virus, to inhibit viral transmission, or to prevent the virus from establishing itself in its host, and to ameliorate or alleviate the symptoms of the disease caused by the viral infection. The treatment is considered therapeutic if there is a reduction in viral load, decrease in mortality and/or morbidity.

IC50 or IC90 (inhibitory concentration 50 or 90) is a concentration of a therapeutic agent, such as an iminosugar, used to achieve 50% or 90% reduction of viral load, respectively.

### Disclosure

The present inventors discovered that certain iminosugars, such as deoxynojirimycin derivatives, can be effective against viruses belonging to the Orthomyxoviridae family, also known as orthomyxoviruses.

In particular, iminosugars can be useful for treating and/or preventing a disease or condition caused by or associated with a virus belonging to the Orthomyxoviridae family.

The Orthomyxoviridae family is a family of RNA viruses that includes five genera: Influenzavirus A, Influenzavirus B, Influenzavirus C, Isavirus and Thogotovirus. The first three genera contain viruses that can cause influenza in vertebrates, including birds, humans and other mammals.

The Influenzavirus A genus includes a single species, which can causes influenza in birds and certain mammals, including humans, pigs, felines, canines and equines.

Influenza A viruses are negative sense, single-stranded, segmented RNA viruses. Several subtypes of Influenza A virus exist, labeled according to an H number (for the type of hemagglutinin) and an N number (for the type of neuraminidase). Currently known 16 different H antigens (H1 to H16) and nine different N antigens (N1 to N9). Serotypes and subtypes of Influenza A include H1N1 Influenza A; H1N2 Influenza A; H2N2 Influenza A; H3N1 Influenza A; H3N2 Influenza A; H3N8 Influenza A; H5N1 Influenza A; H5N2 Influenza A; H5N3 Influenza A; H5N8 Influenza A; H5N9 Influenza A; H5N9 Influenza A; H7N1 Influenza A; H7N2 Influenza A; H7N3 Influenza A; H7N4 Influenza A; H7N7 Influenza A; H9N2 Influenza A; H10N7 Influenza A.

The Influenzavirus B genus includes a single species, which can cause influenza in humans and seals.

The Influenzavirus C genus includes a single species, which can cause influenza in humans and pigs.

In many embodiments, the iminosugar may be N-substituted deoxynojirimycin. In some embodiments, such N-substituted deoxynojirimycin may be a compound of the following formula: 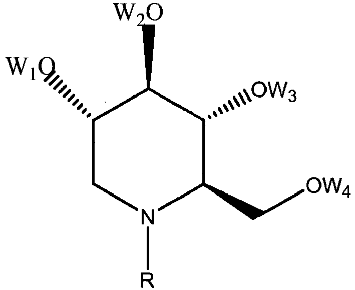 where W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

In some embodiments, R may be selected from substituted or unsubstituted cycloalkyl groups, substituted or unsubstituted aryl groups, or substituted or unsubstituted oxaalkyl groups.

In some embodiments, R may be substituted or unsubstituted oxaalkyl groups comprise from 1 to 16 carbon atoms, from 4 to 12 carbon atoms or from 8 to 10 carbon atoms. The term "oxaalkyl" refers to an alkyl derivative, which can contain from 1 to 5 or from 1 to 3 or from 1 to 2 oxygen atoms. The term "oxaalkyl" includes hydroxyterminated and methoxyterminated alkyl derivatives.

In some embodiments, R may be selected from, but is not limited to -(CH₂)₆OCH₃, -(CH₂)₆OCH₂CH₃, -(CH₂)₆O(CH₂)₂CH₃, -(CH₂)₆O(CH₂)₃CH₃, -(CH₂)₂O(CH₂)₅CH₃, -(CH₂)₂O(CH₂)₆CH₃,;-(CH₂)₂O(CH₂)₇CH₃; -(CH₂)₉-OH; -(CH₂)₉OCH₃.

In certain embodiments, the alkyl group may be a long chain alkyl group, which may be C6-C20 alkyl group; C8-C16 alkyl group; or C8-C10 alkyl group. In some embodiments, R may be a long chain oxaalkyl group, i.e. a long chain alkyl group, which can contain from 1 to 5 or from 1 to 3 or from 1 to 2 oxygen atoms.

In some embodiments, R may have the following formula 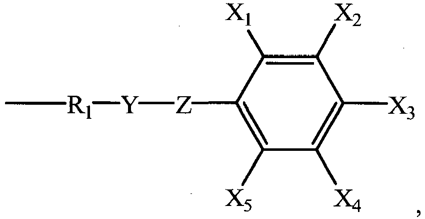 where R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl.

In some embodiments, Z is NH and R₁-Y is a substituted or unsubstituted alkyl group, such as C2-C20 alkyl group or C4-C12 alkyl group or C4-C10 alkyl group.

In some embodiments, X₁ is NO₂ and X₃ is N₃. In some embodiments, each of X₂, X₄ and X₅ is hydrogen.

In some embodiments, the iminosugar may be a DNJ derivative disclosed in U.S. Patent application publication no. 2007/0275998.

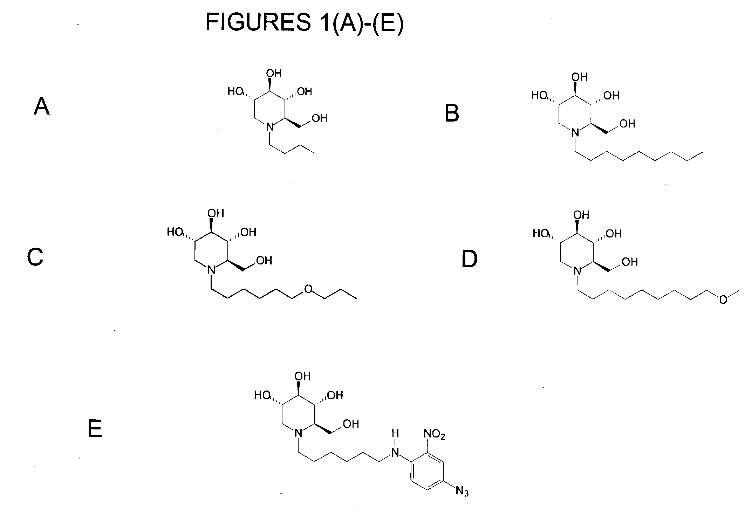

In some embodiments, the iminosugar may be one of the compounds presented in Figure 1. Methods of synthesizing deoxynojirimycin derivatives are disclosed, for example, in U.S.

Patent Nos. 5,622,972, 5,200,523, 5,043,273, 4,994,572, 4,246,345, 4,266,025, 4,405,714, and 4,806,650 and U.S. Patent application publication no. 2007/0275998.

In some embodiments, the iminosugar may be in a form of a salt derived from an inorganic or organic acid. Pharmaceutically acceptable salts and methods for preparing salt forms are disclosed, for example, in Berge et al. (J. Pharm. Sci. 66:1-18, 1977). Examples of appropriate salts include but are not limited to the following salts: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate, and undecanoate.

In some embodiments, the iminosugar may also used in a form of a prodrug. Prodrugs of DNJ derivatives, such as the 6-phosphorylated DNJ derivatives, are disclosed in U.S. Patents nos. 5,043,273 and 5,103,008.

In some embodiments, the iminosugar may be used as a part of a composition, which further comprises a pharmaceutically acceptable carrier and/ or a component useful for delivering the composition to an animal. Numerous pharmaceutically acceptable carriers useful for delivering the compositions to a human and components useful for delivering the composition to other animals such as cattle are known in the art. Addition of such carriers and components to the composition of the invention is well within the level of ordinary skill in the art.

In some embodiments, the pharmaceutical composition may consist essentially of N-substituted deoxynojirimycin, which may mean that the *N*-substituted deoxynojirimycin is the only active ingredient in the composition.

Yet in some embodiments, *N*-substituted deoxynojirimycin may be administered with one or more additional antiviral compounds.

In some embodiments, the treatment or prevention of the disease or condition caused by or associated with a virus belonging to the Orthomyxoviridae family may be performed without administering N-(phosphonoacetyl)-L-aspartic acid to the subject, to whom the iminosugar is being administered. N-(phosphonoacetyl)-L-aspartic acid is disclosed, for example, in U.S. patent no. 5,491,135.

In some embodiments, the treatment or prevention of the disease or condition caused by or associated with a virus belonging to the Orthomyxoviridae family may be performed without administering to the subject a pyrrolizidine compound, such as compounds disclosed in U.S. patent no. 5,021,427 and U.S. patent publication 20070155814.

In some embodiments, the treatment or prevention of the disease or condition caused by or associated with a virus belonging to the Orthomyxoviridae family may be performed without administering to the subj ect australine.

In some embodiments, the iminosugar, such as *N*-substituted deoxynojirimycin, may be used in a liposome composition, such as those disclosed in US publications nos. 2008/0138351 and 2009/0252785 as well as in US application No. 12/732630 filed March 26, 2010.

The iminosugar, such as N-substituted DNJ derivative, may be administered to a cell or an animal affected by a virus. The iminosugar may inhibit morphogenesis of the virus, or it can treat the individual. The treatment may reduce, abate, or diminish the virus infection in the animal.

Animals that can be infected with a virus that belongs to the Orthomyxoviridae family, include vertebrates, such as birds and mammals, including primates, such as humans; felines; equines, and canines.

The amount of iminosugar administered to an animal or to an animal cell to the methods of the invention may be an amount effective to inhibit the morphogenesis of a virus belonging to the Orthomyxoviridae family from the cell. The term "inhibit" as used herein may refer to the detectable reduction and/or elimination of a biological activity exhibited in the absence of the iminosugar. The term "effective amount" may refer to that amount of the iminosugar necessary to achieve the indicated effect. The term "treatment" as used herein may refer to reducing or alleviating symptoms in a subject, preventing symptoms from worsening or progressing, inhibition or elimination of the causative agent, or prevention of the infection or disorder related to the virus belonging to the Orthomyxoviridae family in a subject who is free therefrom.

Thus, for example, treatment of the disease caused by or associated with a virus may include destruction of the infecting agent, inhibition of or interference with its growth or maturation, and neutralization of its pathological effects. The amount of the iminosugar which may be administered to the cell or animal is preferably an amount that does not induce toxic effects which may outweigh the advantages which accompany its administration.

Actual dosage levels of active ingredients in the pharmaceutical compositions may vary so as to administer an amount of the active compound(s) that is effective to achieve the desired therapeutic response for a particular patient.

The selected dose level can depend on the activity of the iminosugar, the route of administration, the severity of the condition being treated, and the condition and prior medical history of the patient being treated. However, it is within the skill of the art to start doses of the compound(s) at levels lower than required to achieve the desired therapeutic effect and to gradually increase the dosage until the desired effect is achieved. If desired, the effective daily dose may be divided into multiple doses for purposes of administration, for example, two to four doses per day. It will be understood, however, that the specific dose level for any particular patient can depend on a variety of factors, including the body weight, general health, diet, time and route of administration and combination with other therapeutic agents and the severity of the condition or disease being treated. The adult human daily dosage may range from between about one microgram to about one gram, or from between about 10 mg and 100 mg, of the iminosugar per 10 kilogram body weight. In some embodiments, a total daily dose may be from 0.1 mg/kg body weight to 100 mg/kg body weight or from 1 mg/kg body weight to 60 mg/kg body weight or from 2 mg/kg body weight to 50 mg/kg body weight or from 3 mg/kg body weight to 30 mg/kg body weight. The daily dose may be administered over one or more administering events over day. For example, in some embodiments, the daily dose may be distributed over two (BID) administering events per day, three administering events per day (TID) or four administering events (QID). In certain embodiments, a single administering event dose ranging from 1 mg/kg body weight to 10 mg/kg body weight may be administered BID or TID to a human making a total daily dose from 2 mg/kg body weight to 20 mg/kg body weight or from 3 mg/kg body weight to 30 mg/kg body weight. Of course, the amount of the iminosugar which should be administered to a cell or animal can depend upon numerous factors well understood by one of skill in the art, such as the molecular weight of the iminosugar and the route of administration. Pharmaceutical compositions that are useful in the methods of the invention may be administered systemically in oral solid formulations, ophthalmic, suppository, aerosol, topical or other similar formulations. For example, it may be in the physical form of a powder, tablet, capsule, lozenge, gel, solution, suspension, syrup, or the like. In addition to the iminosugar, such pharmaceutical compositions may contain pharmaceutically-acceptable carriers and other ingredients known to enhance and facilitate drug administration. Other possible formulations, such as nanoparticles, liposomes, resealed erythrocytes, and immunologically based systems may also be used to administer the iminosugar. Such pharmaceutical compositions may be administered by a number of routes. The term "parenteral" used herein includes subcutaneous, intravenous, intraarterial, intrathecal, and injection and infusion techniques, without limitation. By way of example, the pharmaceutical compositions may be administered orally, topically, parenterally, systemically, or by a pulmonary route.

These compositions may be administered a in a single dose or in multiple doses which are administered at different times. Because the inhibitory effect of the composition upon a virus belonging to the Orthomyxoviridae family may persist, the dosing regimen may be adjusted such that virus propagation is retarded while the host cell is minimally effected. By way of example, an animal may be administered a dose of the composition of the invention once per week, whereby virus propagation is retarded for the entire week, while host cell functions are inhibited only for a short period once per week.

Embodiments described herein are further illustrated by, though in no way limited to, the following working examples.

### Working Examples

### 1. Synthesis ofN-Nonyl DNJ

**Table 1. Materials for NN-DNJ synthesis**

| Name | Amount |
|---|---|
| DNJ | 500 mg |
| Nonanal | 530 mg |
| Ethanol | 100 mL |
| AcOH | 0.5 mL |
| Pd/C | 500 mg |

Procedure: A 50-mL, one-necked, round-bottom flask equipped with a magnetic stirrer was charged with DNJ (500 mg), ethanol (100 mL), nonanal (530 mg), and acetic acid (0.5 mL ) at room temperature. The reaction mixture was heated to 40-45 °C and stirred for 30-40 minutes under nitrogen. The reaction mixture was cooled to ambient temperature and Pd/C was added. The reaction flask was evacuated and replaced by hydrogen gas in a balloon. This process was repeated three times. Finally, the reaction mixture was stirred at ambient temperature overnight. The progress of reaction was monitored by TLC (Note 1). The reaction mixture was filtered through a pad of Celite and washed with ethanol. The filtrate was concentrated *in vacuo* to get the crude product. The crude product was purified by column chromatography (230-400 mesh silica gel). A solvent gradient of methanol in dichloromethane (10-25%) was used to elute the product from the column. All fractions containing the desired product were combined, and concentrated *in vacuo* to give the pure product (420mg). Completion of the reaction was monitored by thin layer chromatography (TLC) using a thin layer silica gel plate; eluent; methanol: dichloromethane = 1:2

### 2. Synthesis of N-7-Oxadecyl DNJ

### 2a. Synthesis of 6-propyloxy-1-hexanol

**Table 2. Materials for synthesis of 6-propyloxy-1-hexanol**

| Name | Amount |
|---|---|
| 1,6-hexanediol | 6.00 g |
| 1-Iodopropane | 8.63 g |
| Potassium tert-butoxide | 5.413 mg |
| THF | 140 mL |

Procedure: a 500-mL, one-necked, round-bottom flask equipped with a magnetic stirrer was charged with 1,6-hexanediol (6.00 g), potassium tert-butoxide (5.413 g) at room temperature. The reaction mixture was stirred for one hour, and then 1-iodopropane (8.63 g) was added. The reaction mixture was heated to 70-80 °C and stirred overnight. The progress of reaction was monitored by TLC (Note 1). After completion of the reaction, water was added to the reaction mixture, and extracted with ethyl acetate (2 x 100 mL). The combined organic layers were concentrated *in vacuo* to get the crude product. The crude product was dissolved in dichloromethane and washed with water, and then brine, dried over sodium sulfate. The organic layer was concentrated *in vacuo* to get the crude product. The crude product was purified by column chromatography using 230-400 mesh silica gel. A solvent gradient of ethyl acetate in hexanes (10-45%) was used to elute the product from the column. All fractions containing the desired pure product were combined and concentrated *in vacuo* to give pure 6-propyloxy-1-hexanol (lot D-1029-048, 1.9 g, 25%) Completion of the reaction was monitored by thin layer chromatography (TLC); (eluent: 60% ethyl acetate in hexanes).

### 2b. Preparation of 6-propyloxy-1-hexanal

**Table 3. Materials for preparation of 6-propyloxy-1-hexanal**

| Name | Amount |
|---|---|
| 6-Propyloxy-1-hexanol | 1.00 g |
| PDC | 4.70 g |
| Celite | 1.00 g |
| NaOAc | 100 mg |
| CH₂Cl₂ | 10 mL |

Procedure: a 50-mL, one-necked, round-bottom flask equipped with a magnetic stirrer was charged with 6-propyloxy-1-hexanol (1.0 g), PDC (4.7 g), dichloromethane (10 mL), Celite (1.0 g), and sodium acetate (100 mg). The reaction mixture was stirred at room temperature under nitrogen for 5 minutes. PDC (4.70 g) was added to the reaction mixture, and stirred overnight. The progress of reaction was monitored by TLC (Note 1). After completion of the reaction, the reaction mixture was directly loaded on the column (230-400 mesh silica gel). A solvent gradient of dichloromethane in ethyl acetate (10-20%) was used to elute the product from the column. All fractions containing the desired pure product were combined and concentrated *in vacuo* to give pure 6-propyloxy-1-hexanal (lot D-1029-050, 710 mg, 71 %). Completion of the reaction was monitored by thin layer chromatography (TLC); (eluent: 60% ethyl acetate in hexanes).

### 2c Synthesis of N-7-Oxadecyl-DNJ

**Table 4. Materials for Synthesis of N-7-Oxadecyl-DNJ**

| Name | Amount |
|---|---|
| DNJ | 500 mg |
| 6-Propyloxy-1-hexanal | 585 mg |
| Pd/C | 125 mg |
| Ethanol | 15 mL |
| Acetic acid | mL |

Procedure: a 50-mL, one-necked, round-bottom flask equipped with a magnetic stirrer was charged with DNJ (500 mg), ethanol (15 mL), 6-propyloxy-1-hexanal (585 mg), and acetic acid (0.1mL) t room temperature. The reaction mixture was heated to 40-45 °C and stirred for 30-40 minutes under nitrogen. The reaction mixture was cooled to ambient temperature and Pd/C was added. The reaction flask was evacuated and replaced by hydrogen gas in a balloon. This process was repeated three times. Finally, the reaction mixture was stirred at ambient temperature overnight. The progress of reaction was monitored by TLC (Note 1). The reaction mixture was filtered through a pad of Celite and washed with ethanol. The filtrate was concentrated *in vacuo* to get the crude product. The crude product was purified by column chromatography (230-400 mesh silica gel). A solvent gradient of methanol in dichloromethane (10-40%) was used to elute the product from the column. All fractions containing the desired product were combined, and concentrated *in vacuo* to give the pure product. (Lot: D-1029-052 (840 mg). Completion of the reaction was monitored by thin layer chromatography (TLC); (eluent: 50% methanol in dichloromethane).

### 3. Synthesis ofN-(9-methoxy)-nonyl DNJ

### 3a Preparation of 9-methoxy-1-nonanol

**Table 5. Materials for preparation of 9-methoxy-1-nonanol**

| Name | Amount |
|---|---|
| 1,9-nonanediol | 10.0 g |
| Dimethyl sulfate | 41.39 g |
| Sodium hydroxide | 5.0g |
| DMSO | 100 mL |

Procedure: a 500-mL, one-necked, round-bottom flask equipped with a magnetic stirrer and stir bar was charged with 1,9-nonanediol (10.00 g, 62.3 mmol) in dimethyl sulfoxide (100 mL) and H₂O (100 mL). To this was added slowly a solution of sodium hydroxide (5.0 g, 125.0 mmol) in H₂O (10 mL) at room temperature. During addition of sodium hydroxide the reaction mixture generated heat and the temperature rose to ∼40 °C. The mixture was stirred for one hour, and then dimethyl sulfate (16.52 g, 131 mmol) was added in four portions while maintaining the temperature of the reaction mixture at ∼ 40°C. The reaction mixture was stirred at room temperature overnight. Progress of the reaction was monitored by TLC (Note 1). TLC monitoring indicated that the reaction was 25 % conversion. At this stage additional dimethyl sulfate (24.78g, 196.44 mmol) was added and the resulting mixture was stirred at room temperature for an additional 24 h. After completion of the reaction, sodium hydroxide (10% solution in water) was added to the reaction mixture to adjust the pH of the solution to 11-13. The mixture was stirred at room temperature for 2 h and extracted with dichloromethane (3 x 100 mL). The combined organic layers were washed with H₂O (200 mL), brine (150 mL), dried over anhydrous sodium sulfate (20 g), filtered and concentrated *in vacuo* to obtain a crude product (14 g). The crude product was purified by column chromatography using 250-400 mesh silica gel. A solvent gradient of ethyl acetate in hexanes (10-50%) was used to elute the product from the column. All fractions containing the desired pure product were combined and concentrated *in vacuo* to give pure 9-methoxy-1-nonanol (lot D-1027-155, 2.38 g, 21.9 %). Completion of the reaction was monitored by thin layer chromatography (TLC) using a thin layer silica gel plate; eluent: 60% ethyl acetate in hexanes.

### 3b Preparation of 9-methoxy-1-nonanal

**Table 6. Materials for preparation of 9-methoxy-1-nonanal**

| Name | Amount |
|---|---|
| 9-methoxy-1-nonanol | 1.0 g |
| PDC | 4.7 g |
| Molecular sieves, 3A | 1.0 g |
| NaOAc | 0.1g |
| CH₂Cl₂ | 10 mL |

Procedure: a 50-mL, one-necked, round-bottom flask equipped with a magnetic stirrer and stir bar was charged with 9-methoxy-nonanol (1.0 g, 5.9 mmol), dichloromethane (10 mL), molecular sieves (1.0 g, 3A), sodium acetate (0.1 g) at room temperature. The reaction mixture was stirred at room temperature under nitrogen for 5 minutes. The reaction mixture was charged with pyridinium dichromate (4.7 g, 12.5 mmol) and stirred overnight. The progress of reaction was monitored by TLC (Note 1). After completion of the reaction, the reaction mixture was filtered through a bed of silica gel (∼15 g). The filtrate was evaporated in *vacuo* to obtain a crude compound. This was purified by column chromatography using silica gel column (250-400 mesh, 40 g). A solvent gradient of ethyl acetate in hexane (10-50%) was used to elute the product from the column. All fractions containing the desired pure product were combined and concentrated *in vacuo* to give pure 9-methoxy-nonanal (lot D-1027-156, 553 mg, 54.4%). Completion of the reaction was monitored by thin layer chromatography (TLC) using a thin layer silica gel plate; eluent: 60% ethyl acetate in hexanes.

### 3c Synthesis of N-(9-methoxy)-nonyl DNJ

**Table 7. Materials for synthesis ofN-(9-methoxy)-nonyl DNJ**

| Name | Amount |
|---|---|
| DNJ | 300 mg |
| 9-methoxy-1-nonanal | 476 mg |
| Pd/C | 200 mg |
| Ethanol | 20 mL |

Procedure: a 50-mL, two-necked, round-bottom flask equipped with magnetic stirrer and a stir bar was charged with DNJ (300 mg, 1.84 mmol), ethanol (20 mL), 9-methoxy-1-nonanal (476 mg, 2.76 mmol) at room temperature. The reaction mixture was stirred for 5-10 minutes under nitrogen and Pd/C was added at room temperature. The reaction mixture was evacuated and was replaced by hydrogen gas using a balloon. This process was repeated three times and then reaction mixture was stirred under atmospheric hydrogen at room temperature. The progress of reaction was monitored by TLC (Note 1). The reaction mixture was filtered through a bed of Celite and was washed with ethanol (20 mL). The filtrate was concentrated *in vacuo* to get a crude product. The crude product was purified by column chromatography using 250-400 mesh silica gel (20 g). A solvent gradient of methanol in ethyl acetate (5-25%) was used to elute the product from the column. All fractions containing the desired pure product were combined, and concentrated *in vacuo* to give an off white solid. The solid was triturated in ethyl acetate (20 mL), filtered and dried in high vacuum to give a white solid [lot: D-1027-158 (165.3 mg, 28.1%). Completion of the reaction was monitored by thin layer chromatography (TLC) using a thin layer silica gel plate; eluent: 50% methanol in dichloromethane.

### 4. Effects of iminosugars against Influenza A virus

Table provides data for inhibition of infectivity of Influenza A virus H3N2 (Hong Kong) for NB-DNJ (UV-1), NN-DNJ (UV-2), N7-O-DNJ (UV-3), N9-DNJ (UV-4) and NAP-DNJ (UV-5).

| Compound | IC90, µM |
|---|---|
| UV-1 | 20 |
| UV-2 | 0.2 |
| UV-3 | 0.2 |
| UV-4 | 0.2 |
| UV-5 | 0.2 |

Procedure. The compounds were screened for inhibition of generation of infectious virus was conducted on the UV compounds at concentrations up to 500 µM. The influenza virus, Influenza A H3N2, Brisbane/10/2007 strain was evaluated for virus inhibition. MDCK cells (Madin Darby canine kidney cell line) obtained from American Type Culture Collection (ATCC, Manassas, Virginia). Cells were cultured in UltraMDCK, supplemented with 2 mM L-glutamine, 1 µg/ml TPCK-treated trypsin and 100 U/ml penicillin, 100 µg/ml streptomycin in cell culture treated 24-well flat bottom plates at 37°C in a 5% CO2 incubator for 24 hr or until 80% confluent prior to assay. Cells were pretreated with compounds in a final concentration of 0.5% DMSO for 1 hr followed by addition of virus inoculums. Three wells per virus are saved for a virus-only control. Only medium is added in exchange for compound in these wells, and virus is added after the initial 1 hr incubation. Three days later virus containing supernatants were collected and effect on reduction of virus yield are tested by assaying frozen and thawed eluates from each well for virus titer by serial dilution onto monolayers of MDCK susceptible cells. The 90% effective concentration (EC90), which is that test drug concentration that inhibits virus yield by 1 log10, is determined from these data.

### Influenza In Vivo Study

UV-4 was administered as a free drug dissolved in acidic water. The compound was given at 100 mg/kg and 10 mg/kg by the oral route (intragastric via oral gavage - IG) twice daily. Balb/c mice received the compound for 10 days. Mice were infected with INFV A H1N1 (strain A/Texas) intranasally with ∼5 LD50 30 minutes following the first iminosugar dose. Animals were monitored for 15 days. Animals were weighed once per day, and given health scores 2X per day. Animals displaying severe illness (as determined by 30% weight loss, extreme lethargy, ruffled coat, or paralysis) were euthanized.

Figure 5 shows effects of 10-day administration of UV-4 on survival of mice infected with influenza A H1N1.

Results: Animals receiving 100mg/kg and 10mg/kg BID showed a 90% survival rate, versus a 30% survival rate in control animals.

Conclusion: These results demonstrate that UV-4 can be used as a host-based antiviral drug to treat influenza A.

### Iminosugar Safety Study

Methods and Discussion: BALB/c and C57/B1/6 mice were given oral suspensions of UV-1, UV-4, UV-5, twice a day for seven days, in 100ul per mouse at 100 and 10 mg/kg (2mg and 0.2 mg/mouse, respectively) 8 hours apart for 7 days, and then monitored for weight loss and general health. After seven days of treatment, the mice did not show any significant signs of weight loss compared to the "vehicle only" control. The results of these experiments are in Figure 6.

When the BALB/c mice were treated with UV-5 at the highest concentration, they displayed signs of diarrhea, red urine, and a ruffled appearance although they did not show signs of weight loss. The C57B1/6 mice displayed these same symptoms but without the ruffled look. These symptoms promptly ceased when treatment was done, and by day 11 (day 4 post compound treatment) the BALB/c mice in these groups looked very healthy.

Conclusions: These compounds have shown to be relatively non-toxic in this mouse model and these concentrations of compound are deemed safe.

### Second Influenza In Vivo Study

Figure 7 presents survival data after H1/N1 (Texas) virus challenge for mice treated with UV-4 versus control mice.

UV-4 was administered to the treated mice as a free drug dissolved in acidic water by the oral route (intragastric via oral gavage - IG) 100mg/kg, TID for 10 days. The control mice received water orally, TID, instead of UV-4. Balb/c mice were used both for the treated mice and the control mice. Each mouse was microchipped for individual identity.

Mice were infected with INFV A H1N1 (strain A/Texas) intranasally witch ∼1 LD90. Animals were monitored for 15 days. Animals were weighed once per day, and given health scores 2X per day. Animals displaying severe illness (as determined by 30% weight loss, extreme lethargy, ruffled coat, or paralysis) were euthanized. The endpoint was considered a death of the animal or a more than 30% weight loss.

The studied mice include the following groups (10 mice per group):
1) 1 hr pre-treatment. These mice received their first UV-4 dose 1 hr before being infected with INFV A H1N1 (strain A/Texas).
2) 24 hr post-treatment. These mice received their first UV-4 dose 24 hr after being infected with INFV A H1N1 (strain A/Texas).
3) 48 hr post treatment. These mice received their first UV-4 48 hr after being infected with INFV A H1N1 (strain A/Texas).
4) 96 hr post treatment. These mice received their first UV-4 96 hr after being infected with INFV A H1N1 (strain A/Texas).

Results: Animals in the 1 hr pre-treatment and 24 hr post-treatment groups demonstrated 100 % survival during the experiment's duration, while mice in the 48 hr post-treatment and 96 hr post-treatment groups demonstrated 90% survival. The survival rate for the control mice was 30%.

Conclusion: These results demonstrate that UV-4 can be used as a host-based antiviral drug to treat and prevent influenza A.

## Claims

1. A compound of the below formula for use in a method of treating and/or preventing in a subject a disease or condition caused by or associated with a virus belonging to the Orthomyxoviridae family: 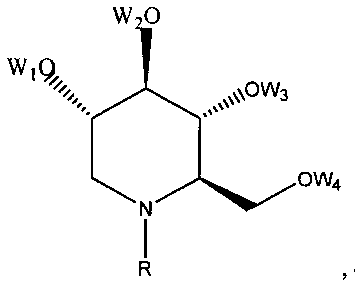 or a pharmaceutically acceptable salt thereof, wherein R is selected from substituted or unsubstituted oxaalkyl groups; substituted or unsubstituted cycloalkyl groups, or substituted or unsubstituted aryl groups; or wherein R is 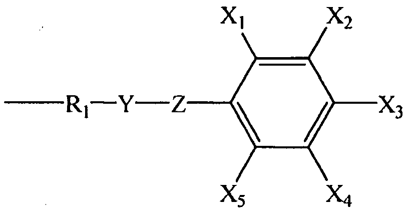
R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl; and wherein W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

2. The compound for use of claim 1, wherein each of W₁, W₂, W₃ and W₄ is hydrogen.

3. The compound for use of claim 1, wherein R is selected from substituted or unsubstituted oxaalkyl groups; substituted or unsubstituted cycloalkyl groups; or substituted or unsubstituted aryl groups.

4. The compound for use of claim 1, wherein R is C6-C12 oxalkyl group.

5. The compound for use of claim 4, wherein R is C8-C10 oxalkyl group.

6. The compound for use of claim 1, wherein said compound is (i) N-(9-Methoxynonyl)deoxynojirimycin or a pharmaceutically acceptable salt thereof; (ii) N-(7-oxadecyl)deoxynojirimycin or a pharmaceutically acceptable salt thereof; or (iii) *N*-(*N*-{4'-azido-2'-nitrophenyl}-6-aminohexyl)deoxynojirimycin or a pharmaceutically acceptable salt thereof.

7. The compound for use of claim 1, wherein R is

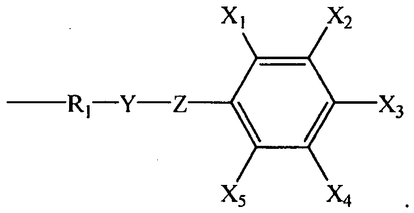

8. The compound for use of claim 7, wherein X₁ is NO₂ and X₃ is N₃.

9. The compound for use of claim 1, wherein each of X₂, X₄ and X₅ is hydrogen.

10. The compound for use of claim 1, wherein the subject is a mammal.

11. The compound for use of claim 10, wherein the subject is a human being.

12. The compound for use of claim 1, wherein the virus is an Influenza virus belonging to the Influenza A, Influenza B or Influenza C genus.

13. The compound for use of claim 12, wherein the virus is Influenza A virus.

14. The compound for use of claim 13, wherein the virus is (i) a H3N2 subtype of the Influenza A virus; or (ii) a H1N1 subtype of the Influenza A virus.

15. The compound for use of claim 14, wherein the virus is a H1N1 subtype of the Influenza A virus and the compound is N-(9-Methoxynonyl)deoxynojirimycin or a pharmaceutically acceptable salt thereof.

## Patentansprüche

1. Verbindung der nachfolgenden Formel zur Verwendung in einem Verfahren zur Behandlung und/oder Vorbeugung einer Krankheit oder eines Zustandes in einem Subjekt, hervorgerufen durch oder im Zusammenhang stehend mit einem Virus, das zu der Orthomyxoviridae-Familie gehört: 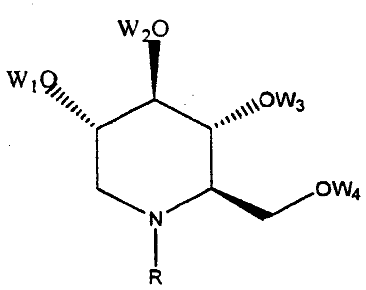 oder ein pharmazeutisch annehmbares Salz davon, wobei R aus substutierten oder unsubstituierten Oxalkylgruppen; substituierten oder unsubstituierten Cycloalkylgruppen oder substituierten oder unsubstituierten Arylgruppen ausgewählt wird; oder wobei R 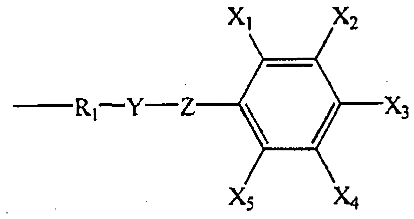 ist;
R₁ eine substituierte oder unsubstituierte Alkylgruppe ist;
X₁₋₅ unabhängig aus H, NO₂, N₃ oder NH₂ ausgewählt werden;
Y nicht vorhanden ist oder eine substituierte oder unsubstituierte von Carbonyl verschiedene C₁-Alkylgruppe ist; und
Z aus einer Bindung oder NH ausgewählt wird; mit der Maßgabe, dass, wenn Z eine Bindung ist, Y nicht vorhanden ist, und mit der Maßgabe, dass, wenn Z NH ist, Y eine von Carbonyl verschiedene substituierte oder unsubstituierte C₁-Alkylgruppe ist; und wobei W₁₋₄ unabhängig aus Wasserstoff, substituierten oder unsubstituierten Alkylgruppen, substituierten oder unsubstituierten Haloalkylgruppen, substituierten oder unsubstituierten Alkanoylgruppen, substituierten oder unsubstituierten Aroylgruppen oder substituierten oder unsubstituierten Haloalkanoylgruppen ausgewählt werden.

2. Verbindung zur Verwendung gemäß Anspruch 1, wobei jedes von W₁, W₂, W₃ und W₄ Wasserstoff ist.

3. Verbindung zur Verwendung gemäß Anspruch 1, wobei R aus substituierten oder unsubstituierten Oxalkylgruppen; substituierten oder unsubstituierten Cycloalkylgruppen; oder substituierten oder unsubstituierten Arylgruppen ausgewählt wird.

4. Verbindung zur Verwendung gemäß Anspruch 1, wobei R eine C6-C12-Oxalkylgruppe ist.

5. Verbindung zur Verwendung gemäß Anspruch 4, wobei R eine C8-C10-Oxalkylgruppe ist.

6. Verbindung zur Verwendung gemäß Anspruch 1, wobei die Verbindung (i) N-(9-Methoxynonyl)deoxynojirimycin oder ein pharmazeutisch annehmbares Salz davon; (ii) N-(7-Oxadecyl)deoxynojirimycin oder ein pharmazeutisch annehmbares Salz davon; oder (iii) N-(N-{4'-Azido-2'-nitrophenyl}-6-aminohexyl)deoxynojirimycin oder ein pharmazeutisch annehmbares Salz davon ist.

7. Verbindung zur Verwendung gemäß Anspruch 1, wobei R 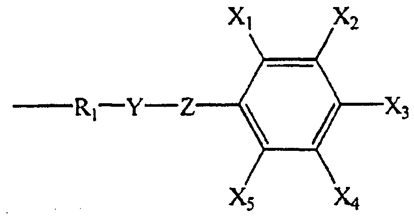 ist.

8. Verbindung zur Verwendung gemäß Anspruch 7, wobei X₁ NO₂ ist und X₃ N₃ ist.

9. Verbindung zur Verwendung gemäß Anspruch 1, wobei jedes von X₂, X₄ und X₅ Wasserstoff ist.

10. Verbindung zur Verwendung gemäß Anspruch 1, wobei das Subjekt ein Säugetier ist.

11. Verbindung zur Verwendung gemäß Anspruch 10, wobei das Subjekt ein Mensch ist.

12. Verbindung zur Verwendung gemäß Anspruch 1, wobei das Virus ein Influenza-Virus ist, welches zu der Influenza A-, Influenza B- oder Influenza C-Gattung gehört.

13. Verbindung zur Verwendung gemäß Anspruch 12, wobei das Virus ein Influenza A-Virus ist.

14. Verbindung zur Verwendung gemäß Anspruch 13, wobei das Virus (i) ein H3N2-Subtyp des Influenza A-Virus oder (ii) ein H1N1-Subtyp des Influenza A-Virus ist.

15. Verbindung zur Verwendung gemäß Anspruch 14, wobei das Virus ein H1N1-Subtyp des Influenza A-Virus ist und die Verbindung N-(9-Methoxynonyl)-deoxynojirimycin oder ein pharmazeutisch annehmbares Salz davon ist.

## Revendications

1. Composé de la formule ci-dessous pour une utilisation dans un procédé de traitement et/ou de prévention chez un sujet, d'une maladie ou d'un état provoqué par ou associé à un virus appartenant à la famille des Orthomyxoviridae : 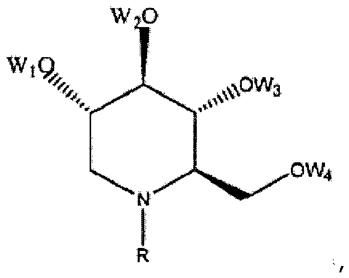 ou un sel pharmaceutiquement acceptable de celui-ci, dans lequel R est choisi parmi les groupes oxaalkyle substitués ou non substitués ; les groupes cycloalkyle substitués ou non substitués, ou les groupes aryle substitués ou non substitués ; ou dans lequel R est 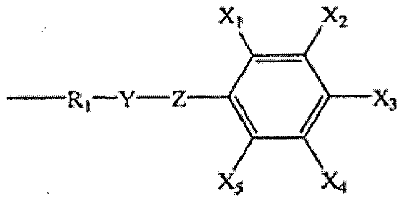
R₁ est un groupe alkyle substitué ou non substitué ;
X₁₋₅ sont indépendamment choisis parmi H, NO₂, N₃, ou NH₂ ;
Y est absent ou est un groupe alkyle en C₁ substitué ou non substitué, autre que le carbonyle ; et
Z est choisi parmi une liaison ou NH ; à condition que lorsque Z est une liaison, Y soit absent, et à condition que lorsque Z est NH, Y soit un groupe alkyle en C₁ substitué ou non substitué, autre que le carbonyle ; et
dans lequel W₁₋₄ sont indépendamment choisis parmi un atome d'hydrogène, les groupes alkyle substitués ou non substitués, les groupes halogénoalkyle substitués ou non substitués, les groupes alcanoyle substitués ou non substitués, les groupes aroyle substitués ou non substitués, ou les groupes halogénoalcanoyle substitués ou non substitués.

2. Composé pour une utilisation selon la revendication 1, dans lequel chacun de W₁, W₂, W₃ et W₄ est un atome d'hydrogène.

3. Composé pour une utilisation selon la revendication 1, dans lequel R est choisi parmi les groupes oxaalkyle substitués ou non substitués ; les groupes cycloalkyle substitués ou non substitués ; ou les groupes aryle substitués ou non substitués.

4. Composé pour une utilisation selon la revendication 1, dans lequel R est un groupe oxalkyle en C6-C12.

5. Composé pour une utilisation selon la revendication 4, dans lequel R est un groupe oxalkyle en C8-C10.

6. Composé pour une utilisation selon la revendication 1, dans lequel ledit composé est (i) la N-(9-méthoxynonyl)désoxynojirimycine ou un sel pharmaceutiquement acceptable de celle-ci ; (ii) la N-(7-oxadécyl)désoxynojirimycine ou un sel pharmaceutiquement acceptable de celle-ci ; ou (iii) la *N*-(*N*-{4'-azido-2'-nitrophényl}-6-aminohexyl)désoxynojirimycine ou un sel pharmaceutiquement acceptable de celle-ci.

7. Composé pour une utilisation selon la revendication 1, dans lequel R est

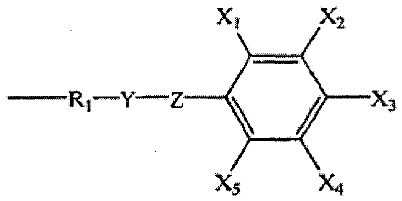

8. Composé pour une utilisation selon la revendication 7, dans lequel X₁ est NO₂ et X₃ est N₃.

9. Composé pour une utilisation selon la revendication 1, dans lequel chacun de X₂, X₄ et X₅ est un atome d'hydrogène.

10. Composé pour une utilisation selon la revendication 1, dans lequel le sujet est un mammifère.

11. Composé pour une utilisation selon la revendication 10, dans lequel le sujet est un être humain.

12. Composé pour une utilisation selon la revendication 1, dans lequel le virus est un virus de la grippe appartenant au genre grippe A, grippe B ou grippe C.

13. Composé pour une utilisation selon la revendication 12, dans lequel le virus est le virus de la grippe A.

14. Composé pour une utilisation selon la revendication 13, dans lequel le virus est (i) un sous-type H₃N₂ du virus de la grippe A ; ou (ii) un sous-type H₁N₁ du virus de la grippe A.

15. Composé pour une utilisation selon la revendication 14, dans lequel le virus est un sous-type H₁N₁ du virus de la grippe A et le composé est la N-(9-méthoxynonyl)désoxynojirimycine ou un sel pharmaceutiquement acceptable de celle-ci.
